# EUROPEAN PATENT APPLICATION

(11) **EP 4 130 272 A1**
(43) Date of publication of application: **08.02.2023**
(21) Application number: 21779277.9
(22) Date of filing: 31.03.2021
(51) Int. Cl.: C12N 15/31, C12Q 1/6813, C12Q 1/6837, C12Q 1/686, C12Q 1/6876

(54) **PRIMER SET AND PROBE FOR DETECTING KLEBSIELLA BACTERIA**

(30) Priority: 31.03.2020 JP 2020063986
(71) Applicant: Denka Company Limited, Tokyo 103-8338 (JP); Toho University, Tokyo 143-8540 (JP)
(72) Inventor: TATEDA, Kazuhiro, Tokyo 143-8541 (JP); MIYATAKE, Yuya, Tokyo 103-8338 (JP); YAMAUCHI, Kazuaki, Tokyo 103-8338 (JP)
(74) Representative: MacLean, Martin Robert
(86) International application number: PCT/JP2021/013800
(87) International publication number: WO 2021/201091

(57) **Abstract**

The present invention provides a primer set for detecting the presence of *Klebsiella variicola* in a specimen, wherein the primer set consists of a first primer and a second primer that target the gyrB gene of *Klebsiella variicola.*

## Description

### Technical Field

The present invention relates to an oligonucleotide for detecting *Klebsiella* spp. bacteria, and more specifically, to an oligonucleotide probe, kit, and method for detecting *Klebsiella variicola* and *Klebsiella pneumoniae* or differentiation between these bacteria.

### Background Art

*Klebsiella spp.* bacteria are gram-negative bacilli that are common in the environment. Among these, *Klebsiella pneumoniae,* also known as the pneumonia bacillus in Japanese, is a resident bacterium in the human intestinal tract and pharynx, and is one of representative bacteria causing opportunistic infections that are pathogenic to humans, such as respiratory infection, urinary tract infection, and bloodstream infection, and is the most frequently observed species of *Klebsiella spp.* bacteria in clinical materials.

In *Klebsiella pneumoniae,* there are resistant strains that possess an enzyme called extended-spectrum β-lactamase (ESBL) that decomposes various antibacterial agents, and is listed as one of carbapenem-resistant Enterobacteriacae (CRE), and the difficulty of its treatment and expansion have become international problems.

**As** a method of detecting infectious disease-causing bacteria such as *Klebsiella pneumoniae,* a culture identification method in which causative bacteria are isolated and cultured, and bacteria are identified based on their biochemical properties and the like are known. So far, it has been found that some bacteria isolated from clinical materials and identified as *Klebsiella pneumoniae* are in fact another species, *Klebsiella variicola,* probably at a proportion of about 10% to 15% (Non-Patent Document 1). *Klebsiella variicola,* a bacterium of the genus *Klebsiella,* is a species related to *Klebsiella pneumoniae,* which was registered as a new species in 2004, and it has been reported that *Klebsiella variicola* is isolated from plants such as bananas and sugar cane and from mycelia in leaf - cutter ant nests, and it is known that *Klebsiella variicola* is not a resident bacteria of humans (Non-Patent Document 1). In existing phenotypic tests, it has been reported that this bacterium tends to differ from *Klebsiella pneumoniae* in some properties (for example, adonitol resolution), but there are currently no definitive indicators, making differentiation difficult.

In addition, it is known that the *Klebsiella spp.* bacteria is currently classified into 14 species and 5 subspecies, and other species that are pathogenic to humans include *Klebsiella aerogenes, Klebsiella oxytoca, Klebsiella planticola* and the like.

As described above, in differentiating *Klebsiella spp.* bacteria contained in clinical materials, many species of *Klebsiella spp.* bacteria should be considered. This differentiation is difficult with existing phenotypic tests, and thus a genetic method such as a sequence analysis, multilocus sequence typing (MLST), or an average nucleotide identity (ANI) method can be used to accurately distinguish *Klebsiella spp.* bacteria contained in clinical materials.

Among these, as a method for differentiating between *Klebsiella pneumoniae* and *Klebsiella variicola,* for example, conventional differentiation has been performed based on gene sequence information such as differentiation (Non-Patent Document 1) by MLST targeting 6 genes such as rpoB, gyrA, mdh, infB, phoE, and nifH genes. However, these methods lack convenience due to the need to perform sequence analysis, and are difficult to use in routine work in the actual medical field.

As a simpler method, a discrimination method by multiplex PCR and gel electrophoresis of 3 genes of blaSHV, blaOKP, and blaLEN, which can be adopted for routine work in the medical field, has been reported (Non-Patent Document 2). However, this method focuses on analysis by gel electrophoresis, with a PCR product length of a minimum of 348 bp and a maximum of 995 bp, and is not suitable for real-time PCR, which is widely used at present, because the product length is too long.

In addition, a method of performing differentiation based on sequence information of 16S rDNA and rpoB genes by combining real-time PCR and Sanger sequencing has also been reported (Non-Patent Document 3). This method also requires sequence analysis for differentiation and lacks simplicity.

### Citation List

### Non-Patent Document

Non-Patent Document 1: Klebsiella variicola, a novel species with clinical and plant associated isolates. Syst Appl Microbiol 27, 27-35
Non-Patent Document 2: A one-step multiplex PCR to identify Klebsiella pneumoniae, Klebsiella variicola, and Klebsiella quasipneumoniae in the clinical routine, Diagnostic Microbiology and Infectious Disease Volume 87, Issue 4, April 2017, Pages 315-317
Non-Patent Document 3: Journal of Microbiological Methods Volume 159, April 2019, Pages 148-156

### Summary

### Technical Problem

In view of the above circumstances, an object of the present invention is to provide an oligonucleotide for detecting *Klebsiella spp.* bacteria (*Klebsiella* spp.), or differentiating these bacteria.

### Solution to Problem

**The** inventors conducted extensive studies and as a result, found a method that can distinguish between *Klebsiella pneumoniae* and *Klebsiella variicola* more easily than previously reported methods by focusing on gyrB gene sequences of *Klebsiella pneumoniae, Klebsiella variicola,* and other *Klebsiella spp.* bacteria, specifically, focusing on a specific position.

Specifically, the present invention encompasses the following inventions.
[1] A primer set for detecting the presence of one or more *Klebsiella spp.* bacteria selected from the group consisting of *Klebsiella variicola* and *Klebsiella pneumoniae* in a specimen, wherein the primer set consists of a first primer and a second primer that target the gyrB gene of *Klebsiella spp.* bacteria.
[2] The primer set according to [1],
   wherein the gyrB gene has a base sequence shown in any of SEQ ID NOs: 1 to 6, or has a base sequence in which one or several bases are deleted, substituted, inserted or added in the base sequence shown in any of SEQ ID NOs: 1 to 6, and encodes a GyrB protein.
[3] The primer set according to [1] or [2],
   wherein the target gyrB gene has a base sequence comprising at least 10 consecutive bases in the base sequence from the position 1887 to the position 2063 of the base sequence shown in any of SEQ ID NOs: 1 to 6.
[4] The primer set according to any one of [1] to [3], which is for detecting the presence of *Klebsiella variicola,*
   wherein the first primer has a base sequence shown in SEQ ID NO: 7 or a base sequence comprising at least 10 consecutive bases in a base sequence in which one or several bases are deleted, substituted, inserted or added in the base sequence shown in SEQ ID NO: 7 and has the same function as the base sequence shown in SEQ ID NO: 7.
[5] The primer set according to any one of [1] to [4], which is for detecting the presence of *Klebsiella variicola,*
   wherein the second primer has a base sequence shown in SEQ ID NO: 8 or a base sequence comprising at least 10 consecutive bases in a base sequence in which one or several bases are deleted, substituted, inserted or added in the base sequence shown in SEQ ID NO: 8 and has the same function as the base sequence shown in SEQ ID NO: 8.
[6] The primer set according to any one of [1] to [5], which is for detecting the presence of *Klebsiella variicola,* and further includes a third primer and a fourth primer targeting the gyrB gene of *Klebsiella pneumoniae.*
[7] The primer set according to any one of [1] to [6],
   wherein the third primer has a base sequence shown in SEQ ID NO: 10 or a base sequence comprising at least 10 consecutive bases in a base sequence in which one or several bases are deleted, substituted, inserted or added in the base sequence shown in SEQ ID NO: 10 and has the same function as the base sequence shown in SEQ ID NO: 10.
[8] The primer set according to any one of [1] to [7],
   wherein the fourth primer has a base sequence shown in SEQ ID NO: 11 or a base sequence comprising at least 10 consecutive bases in a base sequence in which one or several bases are deleted, substituted, inserted or added in the base sequence shown in SEQ ID NO: 11 and has the same function as the base sequence shown in SEQ ID NO: 11.
[9] The primer set according to [1] or [2], which is for detecting the presence of *Klebsiella pneumoniae,*
   wherein the first primer has a base sequence shown in SEQ ID NO: 10 or a base sequence comprising at least 10 consecutive bases in a base sequence in which one or several bases are deleted, substituted, inserted or added in the base sequence shown in SEQ ID NO: 10 and has the same function as the base sequence shown in SEQ ID NO: 10.
[10] The primer set according to any one of [1], [2], and [9],
   wherein the second primer has a base sequence shown in SEQ ID NO: 11 or a base sequence comprising at least 10 consecutive bases in a base sequence in which one or several bases are deleted, substituted, inserted or added in the base sequence shown in SEQ ID NO: 11 and has the same function as the base sequence shown in SEQ ID NO: 11.
[11] The primer set according to any one of [1], [2], [9], and 10, further including
   a third primer and a fourth primer that target the gyrB gene of *Klebsiella variicola.*
[12] The primer set according to any one of [1], [2], and [9] to [11],
   wherein the third primer has a base sequence shown in SEQ ID NO: 7 or a base sequence comprising at least 10 consecutive bases in a base sequence in which one or several bases are deleted, substituted, inserted or added in the base sequence shown in SEQ ID NO: 7 and has the same function as the base sequence shown in SEQ ID NO: 7.
[13] The primer set according to any one of [1], [2], and [9] to [12],
   wherein the fourth primer has a base sequence shown in SEQ ID NO: 8 or a base sequence comprising at least 10 consecutive bases in a base sequence in which one or several bases are deleted, substituted, inserted or added in the base sequence shown in SEQ ID NO: 8 and has the same function as the base sequence shown in SEQ ID NO: 8.
[14] The primer set according to any one of [1] to [13],
   wherein the primers are labeled with biotin.
[15] A probe for detecting the presence of one or more *Klebsiella spp.* bacteria selected from the group consisting of *Klebsiella variicola* and *Klebsiella pneumoniae* in a specimen,
   wherein the probe targets the gyrB gene of *Klebsiella spp.* bacteria.
[16] The probe according to [15],
   wherein the gyrB gene has a base sequence shown in any of SEQ ID NOs: 1 to 6, or has a base sequence in which one or several bases are deleted, substituted, inserted or added in the base sequence shown in any of SEQ ID NOs: 1 to 6, and encodes a GyrB protein.
[17] The probe according to [15] or [16],
   wherein the target gyrB gene has a base sequence comprising at least 10 consecutive bases in the base sequence from the position 1887 to the position 2063 of the base sequence shown in any of SEQ ID NOs: 1 to 6.
[18] The probe according to any one of [15] to [17], which has a base sequence shown in SEQ ID NO: 9 or a base sequence comprising at least 8 consecutive bases in a base sequence in which one or several bases are deleted, substituted, inserted or added in the base sequence shown in SEQ ID NO: 9 and has the same function as the base sequence shown in SEQ ID NO: 9.
[19] A kit including the primer set according to any one of [1] to [14], and the probe according to any one of [15] to [18].
[20] A method for detecting the presence of *Klebsiella variicola* in a specimen, and in which the primer set according to any one of [1] to [9] is used.
[21] The method according to [20], including
   a step of differentiating between *Klebsiella variicola* and other *Klebsiella spp.* bacteria using first and second primers.
[22] The method according to [20] or [21],
   wherein the probe according to any one of [15] to [18] is additionally used.
[23] The method according to any one of [20] to [22],
   wherein the *Klebsiella spp.* bacteria distinguished from *Klebsiella pneumoniae* is one, two or more selected from the group consisting of *Klebsiella pneumoniae; Klebsiella michiganensis; Klebsiella quasipneumoniae*; *Klebsiella aerogenes; Klebsiella planticola; Klebsiella granulomatis; Klebsiella grimontii;* and *Klebsiella oxytoca.*
[24] The method according to any one of [20] to [23],
   wherein the *Klebsiella spp.* bacteria distinguished from *Klebsiella pneumoniae* is *Klebsiella pneumoniae,* and a fourth primer, a fifth primer and/or a probe that target the gyrB gene of *Klebsiella pneumoniae* are additionally used.
[25] A method for detecting the presence of *Klebsiella pneumoniae* in a specimen, and in which the primer set according to any one of [1], [2], and [9] to [14] is used.
[26] The method according to [25], including
   a step of differentiating between *Klebsiella pneumoniae* and other *Klebsiella spp*. bacteria using first and second primers.
[27] The method according to [26],
   wherein the probe according to any one of [15] to [18] is additionally used.
[28] The method according to any one of [25] to [27],
   wherein the *Klebsiella spp.* bacteria distinguished from *Klebsiella pneumoniae* is one, two or more selected from the group consisting of *Klebsiella variicola; Klebsiella michiganensis; Klebsiella quasipneumoniae*; *Klebsiella aerogenes; Klebsiella planticola; Klebsiella granulomatis; Klebsiella grimontii;* and *Klebsiella oxytoca.*
[29] The method according to any one of [25] to [28],
   wherein the *Klebsiella spp.* bacteria distinguished from *Klebsiella pneumoniae* is *Klebsiella variicola,* and a fourth primer, a fifth primer and/or a probe that target the gyrB gene of *Klebsiella variicola* are additionally used.

### Advantageous Effects of Invention

According to oligonucleotides of the present invention, it is possible to distinguish between *Klebsiella variicola* and/or *Klebsiella pneumoniae* and other *Klebsiella spp.* bacteria more easily. In addition, the oligonucleotides of the present invention can exhibit an excellent differentiating ability in comparison with other primers and probes that target the same gyrB genes.

### Brief Description of Drawings

Fig. 1 shows the multiple alignment results of some gyrB genes of gyrB genes (SEQ ID NOs: 1, 2, and 3) of *K. variicola* strain DSM 15968, strain MGH 20, and strain X39, and gyrB genes (SEQ ID NOs: 4, 5, and 6) of *K. pneumoniae* strain NU-CRE047, strain CHS105, and strain KPNIH33.
Fig. 2 shows the results of melting curve analysis of amplification products obtained using primer sets of SEQ ID NOs: 7, 8, 10 and 11, and the solid line indicates *K. variicola* strain JCM 12419, the dashed line indicates *K. pneumoniae* strain JCM 1662^{T}, and the one-dot dashed line indicates the results of a control (water).
Fig. 3 shows the results of detection on a substrate using DNA probes targeting genome DNA of *K. variicola* and *K. pneumoniae.* The vertical axis represents the fluorescence intensity measured in pCode.
Fig. 4 shows the graph obtained by deleting the result of a negative control (water) from the results in Fig. 3. The vertical axis represents the fluorescence intensity measured in pCode.
Fig. 5 shows the results of detection on a substrate using DNA probes targeting clinical specimens. The vertical axis represents the fluorescence intensity measured in pCode.
Fig. 6 shows the multiple alignment results (1 to 301 bp) of some gyrB genes (SEQ ID NOs: 1, 2, and 3) of *K. variicola* strain DSM 15968, strain MGH 20, and strain X39.
Fig. 7 shows the multiple alignment results (300 to 600 bp) of some gyrB genes (SEQ ID NOs: 1, 2, and 3) of *K. variicola* strain DSM 15968, strain MGH 20, and strain X39.
Fig. 8 shows the multiple alignment results (701 to 1000 bp) of some gyrB genes (SEQ ID NOs: 1, 2, and 3) of *K. variicola* strain DSM 15968, strain MGH 20, and strain X39.
Fig. 9 shows the multiple alignment results (1601 to 1900 bp) of some gyrB genes (SEQ ID NOs: 1, 2, and 3) of *K. variicola* strain DSM 15968, strain MGH 20, and strain X39.
Fig. 10 shows the multiple alignment results (2051 to 2350 bp) of some gyrB genes (SEQ ID NOs: 1, 2, and 3) of *K. variicola* strain DSM 15968, strain MGH 20, and strain X39.
Fig. 11 shows the fluorescence measurement results using assay sets 1 to 6.

### Description of Embodiments

Hereinafter, embodiments (hereinafter referred to as "the present embodiment") of the present invention will be described, but the scope of the present invention is not limited to the interpretation of the following embodiments.

### (Primer set)

In a first embodiment, a primer set for detecting the presence of one or more *Klebsiella spp.* bacteria selected from the group consisting of *Klebsiella variicola* and *Klebsiella pneumoniae* in a specimen is provided. The primer set includes a first primer and a second primer that target the gyrB gene of *Klebsiella spp.* bacteria.

The gyrB gene encodes the GyrB protein that is a subunit of DNA gyrase.

The specimen is not particularly limited as long as the presence of *Klebsiella* spp. bacterial strains including *Klebsiella variicola* or *Klebsiella pneumoniae* is suspected, and examples thereof include body fluids such as human or non-human blood, serum, plasma, urine, feces, saliva, sputum, interstitial fluid, cerebrospinal fluid, and swabs and dilutions thereof, and blood, serum, plasma, urine, feces, cerebrospinal fluids or dilutions thereof are preferable. The specimen may be foodstuffs, soil, plants or the like.

Examples of the gyrB gene of *Klebsiella variicola* include those which: have a base sequence shown in any of SEQ ID NOs: 1 to 3; or have a base sequence in which one or several bases are deleted, substituted, inserted or added in the base sequence shown in any of SEQ ID NOs: 1 to 3, and encode the GyrB protein.

Examples of the gyrB gene of *Klebsiella pneumoniae* include those which: have a base sequence shown in any of SEQ ID NOs: 4 to 6; or have a base sequence in which one or several bases are femcdeleted, substituted, inserted or added in the base sequence shown in any of SEQ ID NOs: 4 to 6, and encode the GyrB protein.

For example, the first primer as a forward primer may have any sequence as long as it can hybridize with a base sequence shown in any of SEQ ID NOs: 1 to 6 or a base sequence in which one or several bases are deleted, substituted, inserted or added in the base sequence shown in any of SEQ ID NOs: 1 to 6 under stringent conditions.

**Similarly,** the second primer as a reverse primer may have any sequence as long as it can hybridize with a complementary strand of a base sequence shown in any of SEQ ID NOs: 1 to 6 or a base sequence in which one or several bases are deleted, substituted, inserted or added in a complementary strand of a base sequence shown in any of SEQ ID NOs: 1 to 6 under stringent conditions.

**As** used herein, "stringent conditions" means conditions in which a so-called specific hybrid is formed and a non-specific hybrid is not formed, and for example, can be appropriately determined based on information such as the length of the base sequence. For example, stringent conditions can be set with reference to Molecular Cloning (Fourth Edition, Cold Spring Harbor Laboratory Press, New York). The stringent conditions can be called conditions in which polynucleotides having a high homology (preferably, identity), for example, polynucleotides having a homology of 70% or more, preferably 80% or more, more preferably 90% or more, still more preferably 95% or more, and particularly preferably 99% or more are hybridized, and polynucleotides having a lower homology are not hybridized.

Specific stringent conditions are conditions including incubating a specimen having a base sequence complementary to a desired base sequence and suspected of having *Klebsiella variicola* in a hybridization solution (50% formamide, 10×SSC (0.15 M NaCl, 15 mM sodium citrate, pH 7.0), a 5× Denhardt's solution, 1% SDS, 10% dextran sulfate, 10 µg/ml of denatured Salmon Sperm DNA, and a 50 mM phosphate buffer (pH 7.5)) at about 42°C to about 50°C, and then washing using 0.1×SSC, 0.1% SDS at about 65°C to about 70°C.

Primers and probes are designed so that they do not cause a cross reaction with sequences of other related species, but can be appropriately changed according to the system used for detecting an amplification product and the like, and other conditions. When an amplification product having a length of 100 to 200 bases is generated, for example, it is preferable to target a base sequence from the position 1887 to the position 2063 of the base sequence shown in any of SEQ ID NOs: 1 to 6. The primer is designed to have a base sequence comprising at least 10 consecutive bases among base sequences constituting such a target. It is preferable to perform design so that there is a mismatch with *Klebsiella spp.* bacteria other than *Klebsiella variicola* at the end of the primer, particularly, at the 3' end.

As an example of the first primer targeting the gyrB gene of *Klebsiella variicola,* one having the following base sequence shown in SEQ ID NO: 7 or one having a base sequence comprising at least 10 consecutive bases in a base sequence in which one or several bases are deleted, substituted, inserted or added in the base sequence shown in SEQ ID NO: 7 and having the same function as the base sequence shown in SEQ ID NO: 7 may be exemplified.
5'-GGTGAACACCCTGGTTTC-3' (SEQ ID NO: 7)

As an example of the second primer targeting the gyrB gene of *Klebsiella variicola,* one having the following base sequence shown in SEQ ID NO: 8 or one having a base sequence comprising at least 10 consecutive bases in a base sequence in which one or several bases are deleted, substituted, inserted or added in the base sequence shown in SEQ ID NO: 8 and having the same function as the base sequence shown in SEQ ID NO: 8 may be exemplified.
5'-CGATATTCCGGCCCCATG-3' (SEQ ID NO: 8)

As an example of the first primer targeting the gyrB gene of *Klebsiella pneumoniae,* one having the following base sequence shown in SEQ ID NO: 7 or one having a base sequence comprising at least 10 consecutive bases in a base sequence in which one or several bases are deleted, substituted, inserted or added in the base sequence shown in SEQ ID NO: 7 and having the same function as the base sequence shown in SEQ ID NO: 7 may be exemplified.
5'-GGTGAACACCCTGGTCTC-3' (SEQ ID NO: 10)

As an example of the second primer targeting the gyrB gene of *Klebsiella pneumoniae,* one having the following base sequence shown in SEQ ID NO: 8 or one having a base sequence comprising at least 10 consecutive bases in a base sequence in which one or several bases are deleted, substituted, inserted or added in the base sequence shown in SEQ ID NO: 8 and having the same function as the base sequence shown in SEQ ID NO: 8 may be exemplified.
5'-GATATTCCGGCCCCATAATG-3' (SEQ ID NO: 11)

The length of the first and second primers is arbitrary, and can be, for example, appropriately set to be within a range of about 10 bases to about 35 bases, and is preferably at least 10 bases or more.

The ratio of the first primer and the second primer is not particularly limited, and may be the same as or different from each other. When the primer set is used in an asymmetric PCR method, the concentration of one primer can be adjusted to be higher than the concentration of the other primer.

The first and second primers can specifically amplify all or some regions of *Klebsiella variicola* or the gyrB gene of *Klebsiella pneumoniae,* thus enabling differentiation from other *Klebsiella spp.* bacterial strains. Alternatively, the amplification products of the first and second primers are subjected to melting curve analysis, the melting temperature thereof is compared with the melting temperature of the amplification products of other primer sets, and thus *Klebsiella variicola* or *Klebsiella pneumoniae* can be distinguished from other *Klebsiella spp.* bacterial strains.

Examples of other *Klebsiella spp.* bacterial strains include *Klebsiella michiganensis; Klebsiella quasipneumoniae*; *Klebsiella aerogenes; Klebsiella planticola*; *Klebsiella granulomatis*; *Klebsiella grimontii*; and *Klebsiella oxytoca,* but the bacterial strains contained in the specimen are not limited thereto. In addition, one, two or more species of *Klebsiella spp.* bacterial strains may be used.

The primer set may be a combination of the first and second primers for *Klebsiella variicola* and the first and second primers for *Klebsiella pneumoniae,* and may comprise these first and second primers alone or in combination, or may comprise other primers, for example, additional primers such as third, fourth, fifth, and sixth primers. The detection sensitivity can be improved by using an appropriate additional primer. As such primers, for example, primers for detecting other *Klebsiella spp.* bacterial strains expected to be contained in the specimen and the like are assumed.

As an example of the third primer targeting the gyrB gene of *Klebsiella pneumoniae* that can be used to detect or distinguish *Klebsiella variicola,* one having a base sequence shown in SEQ ID NO: 10 or one having a base sequence comprising at least 10 consecutive bases in a base sequence in which one or several bases are deleted, substituted, inserted or added in the base sequence shown in SEQ ID NO: 10 and having the same function as the base sequence shown in SEQ ID NO: 10 may be exemplified.

As an example of the fourth primer targeting the gyrB gene of *Klebsiella pneumoniae* that can be used to detect or distinguish *Klebsiella variicola,* one having a base sequence shown in SEQ ID NO: 11 or one having a base sequence comprising at least 10 consecutive bases in a base sequence in which one or several bases are deleted, substituted, inserted or added in the base sequence shown in SEQ ID NO: 11 and having the same function as the base sequence shown in SEQ ID NO: 11 may be exemplified.

As an example of the third primer targeting the gyrB gene of *Klebsiella variicola* that can be used to detect or distinguish *Klebsiella pneumoniae,* one having a base sequence shown in SEQ ID NO: 7 or one having a base sequence comprising at least 10 consecutive bases in a base sequence in which one or several bases are deleted, substituted, inserted or added in the base sequence shown in SEQ ID NO: 7 and having the same function as the base sequence shown in SEQ ID NO: 7 may be exemplified.

As an example of the fourth primer targeting the gyrB gene of *Klebsiella variicola* that can be used to detect or distinguish *Klebsiella pneumoniae,* one having a base sequence shown in SEQ ID NO: 8 or one having a base sequence comprising at least 10 consecutive bases in a base sequence in which one or several bases are deleted, substituted, inserted or added in the base sequence shown in SEQ ID NO: 8 and having the same function as the base sequence shown in SEQ ID NO: 8 may be exemplified.

According to the nucleic acid amplification reaction used in the primer, it is possible to detect the presence of *Klebsiella variicola* and/or *Klebsiella pneumoniae* contained in the specimen.

Nucleic acid amplification methods include a PCR method, and examples thereof include a multiplex PCR, a loop-mediated isothermal amplification (LAMP) method, an isothermal and chimeric primer-initiated amplification of nucleic acids (ICAN) method, a rolling circle amplification (RCA) method, a ligase chain reaction (LCR) method, and a strand displacement amplification (SDA) method. When it is expected that a large number of *Klebsiella spp.* bacteria are contained in the specimen, a multiplex PCR is preferable because a plurality of bacteria can be comprehensively and simultaneously detected.

Regarding a method of detecting an amplification product, known methods such as an electrophoresis method using polyacrylamide or agarose gel can be performed. For example, in electrophoresis, the presence of an amplification product can be identified based on the mobility of the amplification product with respect to the mobility of a marker having a known molecular weight.

A label that can specifically recognize an amplification product may be used for detection of the amplification product after the nucleic acid amplification reaction. Examples of such labels include a fluorescent dye, biotin, and digoxigenin. When fluorescence is used as the label, the fluorescence can be detected using a fluorescence microscope, a fluorescence plate reader or the like.

### (Probe)

In a second embodiment, a probe for detecting the presence of one or more *Klebsiella spp.* bacteria selected from the group consisting of *Klebsiella variicola* and *Klebsiella pneumoniae* in a specimen is provided. The probe can target the gyrB gene of *Klebsiella spp.* bacteria.

The probe is preferably designed so that its interior contains a mismatch with respect to other *Klebsiella spp.* bacteria. Thereby, the base length of the full-match part during hybridization can be shortened, the specificity can be improved, and false positives can be reduced.

**As** an example of the probe that can be used to detect or distinguish *Klebsiella variicola,* one having the following base sequence shown in SEQ ID NO: 9 or one having a base sequence comprising at least 8 consecutive bases in a base sequence in which one or several bases are deleted, substituted, inserted or added in the base sequence shown in SEQ ID NO: 9 and having the same function as the base sequence shown in SEQ ID NO: 9 may be exemplified.
5'-GTTGCAGCAGTTTGAG-3' (SEQ ID NO: 9)

**As** an example of the probe that can be used to detect or distinguish *Klebsiella pneumoniae,* one having the following base sequence shown in SEQ ID NO: 12 or one having a base sequence comprising at least 8 consecutive bases in a base sequence in which one or several bases are deleted, substituted, inserted or added in the base sequence shown in SEQ ID NO: 12 and having the same function as the base sequence shown in SEQ ID NO: 12 may be exemplified.
5'-GAACTGCAGCATTTTG-3' (SEQ ID NO: 12)

**The** probe may be bound to a solid-phase component such as beads. Examples of such a solid-phase component include beads suitable for multiplex assays, for example, beads having a unique analog code identifier for storing information for multiplex assays as disclosed in WO2018052464A1.

### (Kit)

In a third embodiment, a kit including a primer set and a probe for detecting the presence of one or more *Klebsiella spp.* bacteria selected from the group consisting of *Klebsiella variicola* and *Klebsiella pneumoniae* in a specimen is provided.

The kit may comprise a reagent used for detecting one or more *Klebsiella spp.* bacteria selected from the group consisting of *Klebsiella variicola* and *Klebsiella pneumoniae,* for example, a reagent for performing an amplification reaction. The reagent for performing an amplification reaction contains, for example, a buffer solution, salts, primers, deoxyribonucleotides, and thermostable DNA polymerase. The content and reagent of the kit can be appropriately determined by those skilled in the art according to the amplification method, and the kit may further comprise a solid-phase component for hybridization with the amplification product obtained using the primer or the probe.

The kit may further comprise primers and probes for detecting other *Klebsiella spp.* bacterial strains expected to be contained in the specimen.

### (Detection method)

In a fourth embodiment, a method for detecting the presence of one or more *Klebsiella spp.* bacteria selected from the group consisting of *Klebsiella variicola* and *Klebsiella pneumoniae* in a specimen is provided. The primer set of SEQ ID NOs: 7 and 8 can be used to detect the presence of *Klebsiella variicola,* and the primer set of SEQ ID NOs: 10 and 11 can be used to detect the presence of *Klebsiella pneumoniae.*

It is preferable to combine the probe with the primer set. For example, when the primer of SEQ ID NOs: 7 and 8 and the probe of SEQ ID NO: 9 are used in combination, *Klebsiella variicola* can be distinguished from other *Klebsiella spp.* bacteria with higher accuracy. The primer set and probe for detecting *Klebsiella pneumoniae* may be used in combination.

In order to distinguish *Klebsiella pneumoniae* from other *Klebsiella spp.* bacteria with high accuracy, for example, it is preferable to use the primer of SEQ ID NOs: 10 and 11 and the probe of SEQ ID NO: 12 in combination. The primer set and probe for detecting *Klebsiella variicola* may be used in combination.

The specimen may be additionally subjected to whole genome analysis, and average nucleotide identity (ANI) analysis to determine differences in detected bacteria.

The present invention will be described below in detail with reference to examples, but the present invention is not limited thereto.

### [Examples]

### Example 1: detection of K. variicola and K. pneumoniae according to real-time PCR targeting genome DNA

### Design of primer and probe

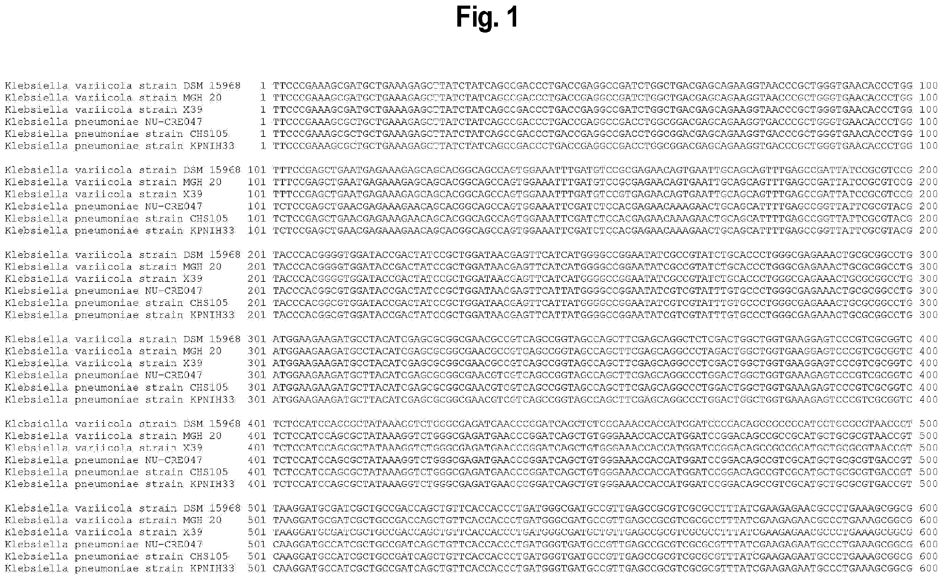

From the multiple alignment results of some gyrB genes of gyrB genes (SEQ ID NOs: 1, 2, and 3) of *K. variicola* strain DSM 15968, strain MGH 20, and strain X39 shown in Fig. 1 and gyrB genes (SEQ ID NOs: 4, 5, and 6) of *K. pneumoniae* strain NU-CRE047, strain CHS105, and strain KPNIH33, the primer and probe of SEQ ID NOs: 7 to 12 were designed.

**[Table 1]**

| | |
|---|---|
| SEQ ID NO: 7: K. variicola primer | GGTGAACACCCTGGTTTC |
| SEQ ID NO: 8: K. variicola primer | CGATATTCCGGCCCCATG |
| SEQ ID NO: 9: K. variicola probe | GTTGCAGCAGTTTGAG |
| SEQ ID NO: 10: K. pneumoniae primer | GCTGAACACCCTGGTCTC |
| SEQ ID NO: 11: K. pneumoniae primer | GATATTCCGGCCCCATAATG |
| SEQ ID NO: 12: K. pneumoniae probe | GAACTGCAGGATTTTG |

### Nucleic acid amplification and melting curve analysis

Amplification by real-time PCR was performed using the genome DNA extracted from *K. variicola* strain JCM 12419 and *K. pneumoniae* strain JCM 1662^{T} as a template, and the primers of SEQ ID NOs: 7, 8, 10, and 11. The reagent used for amplification was TaKaRa Multiplex Assay Kit Ver. 2 (commercially available from Takara Bio Inc.), and EvaGreen (commercially available from biotium) was used as an intercalator dye. Each primer was added at a final concentration of 0.2 µM, the template genome DNA was added at 10 µg/L, a 2×Reaction buffer in TaKaRa Multiplex Assay Kit Ver. 2 was 1×, 1U of Multiplex Enzyme Mix was used, and the template DNA and the like were mixed to prepare a PCR reaction solution. Light Cycler 480 System II (commercially available from Roche Diagnostics) was used as the measurement device.

Amplification reaction conditions used are as follows.
94°C: 30 seconds
60°C: 60 seconds->94°C: 30 seconds (45 cycles)
72°C: 600 seconds

In addition, the amplification product was subjected to melting curve analysis under the following conditions.
95°C: 30 seconds
60°C->95°C: temperature was raised at 0.11°C/sec

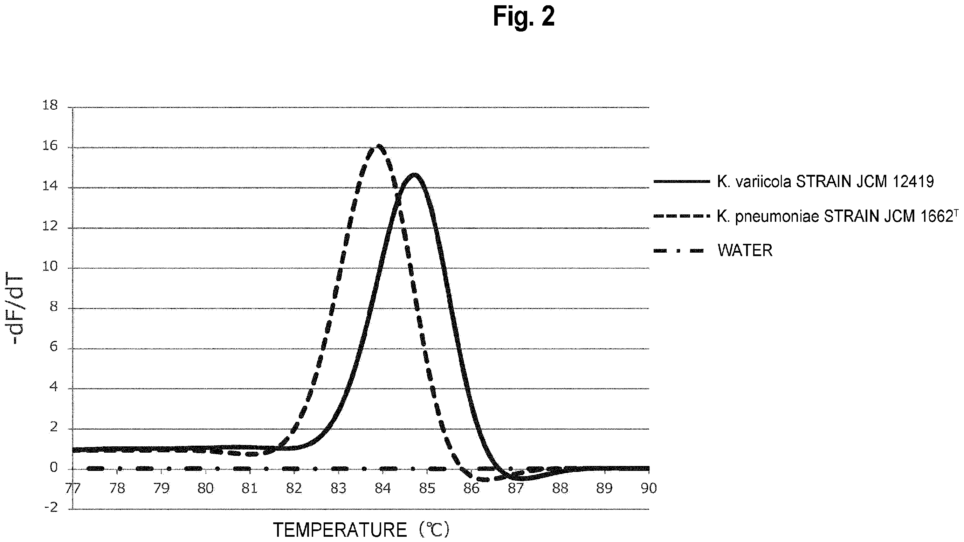

Fig. 2 shows the measurement results of melting curve analysis. The melting temperatures of the amplification products of *K. variicola* and *K. pneumoniae* were 84.7°C and 83.8°C, respectively, with a difference of about 1 °C. From this, it was found that, when the primers of SEQ ID NOs: 7, 8, 10, and 11 were used, it was possible to amplify the target nucleic acid component, and furthermore, it was possible to perform identification according to the melting temperature of the amplification product.

It should be noted that, in this example, notably, the expensive TaqMan Probe, which is often used for luminescent substances in this region, was not used, identification was possible with an inexpensive intercalator dye, and the example excelled in terms of the cost required for the experiment.

### Example 2: detection on substrate using DNA probe targeting genome DNA of K. variicola and K. pneumoniae

### Nucleic acid amplification and detection

Amplification by PCR was performed using the genome DNA extracted from *K. variicola* strain JCM 12419 and *K. pneumoniae* strain JCM 1662^{T} as a template, and the primer sets of SEQ ID NOs: 7, 8, 10, and 11, and the hybridization reaction and labeling on the substrate, and fluorescence measurement were performed. In this case, primers in which the 5' ends of SEQ ID NOs: 8 and 11 were modified with biotin via a linker were used.

TaKaRa Multiplex Assay Kit Ver. 2 (commercially available from Takara Bio Inc.) was used for amplification. Each primer was added at a final concentration of 0.2 µM and the template genome DNA was added at 10 µg/L, a 2×Reaction buffer in TaKaRa Multiplex Assay Kit Ver. 2 was 1×, 1U of Multiplex Enzyme Mix was used, and the template DNA and the like were mixed to prepare a PCR reaction solution. T100 Thermal Cycler (commercially available from Bio-Rad) was used as the nucleic acid amplification device. After the amplification reaction was completed, the heat denaturation reaction was performed with the same instrument before performing the hybridization reaction on the substrate.

For the reagent used for the hybridization reaction on the substrate, a solution containing a substrate 1 on which the probe of SEQ ID NO: 9 was immobilized and a substrate 2 on which the probe of SEQ ID NO: 12 was immobilized, a saline-sodium-phosphate-EDTA Buffer (SSPE-buffer), and a heat-denatured PCR reaction solution were mixed.

Streptavidin - Phycoerythrin (commercially available from PlexBio) was used as a reagent for labeling. For the hybridization reaction and labeling on the substrate, IntelliPlex 1000 πCode Processor (commercially available from PlexBio) was used as an instrument. PlexBio (registered trademark) 100 Fluorescent Analyzer (commercially available from PlexBio) was used as the fluorescence measurement device.

**The** reaction conditions used are as follows.
Amplification reaction conditions
   94°C: 30 seconds
   60°C: 60 seconds->94°C: 30 seconds (30 cycles)
   72°C: 600 seconds
Heat denaturation conditions
   95°C: 5 minutes-> rapid cooling to 4°C
Hybridization conditions
   37°C: 20 minutes incubation
Labeling conditions
   37°C: 10 minutes incubation

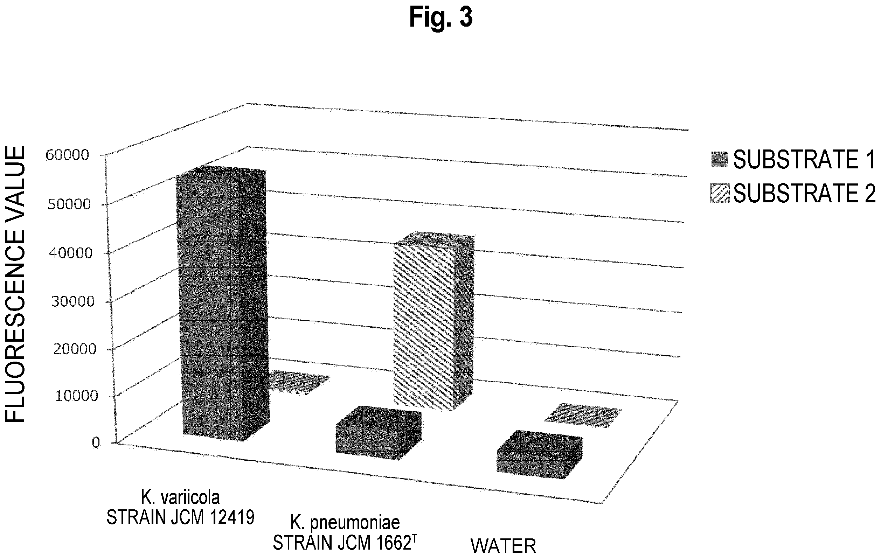

Fig. 3 shows the measurement results. When *K. variicola* strain JCM 12419 was used as a template, a significant fluorescence was observed on the substrate 1, and almost no fluorescence was observed on the substrate 2. In addition, when *K*. *pneumoniae* strain JCM 1662^{T} was used as a template, weak fluorescence was observed on the substrate 1 and significant fluorescence was observed on the substrate 2. In addition, when water (negative control) was used as a template, weak fluorescence was observed on the substrate 1, and almost no fluorescence was observed on the substrate 2.

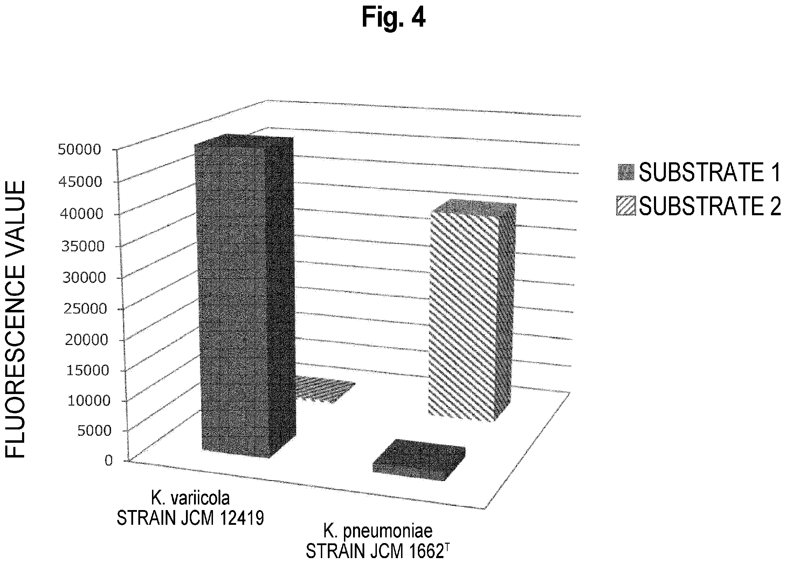

Based on this, it can be determined that the background value of the substrate 1 was high, and the results obtained when the value of the negative control was subtracted from the results in Fig. 3 are shown in Fig. 4.

By subtracting the negative control, when *K. pneumoniae* strain JCM 1662^{T} was used as a template, almost no fluorescence was observed on the substrate 1, and significant fluorescence was observed on the substrate 2.

From this, by using the primers of SEQ ID NOs: 7, 8, 10, and 11 and the probes of SEQ ID NO: 9 and SEQ ID NO: 12, it was expected that differentiation of *K. variicola* and *K. pneumoniae,* which could not be distinguished in the related art, would be possible.

### Example 3: detection on substrate using DNA probe targeting clinical specimen (blood culture solution) 5 specimens

### Nucleic acid amplification and detection

In the culture identification method, for Specimens 1 and 2 identified as *K. pneumoniae,* Specimen 3 identified as *K. aerogenes,* Specimen 4 identified as *K. oxytoca,* and Specimen 5 identified as *K. planticola,* using QIAamp BiOstic Bacteremia DNA Kit (commercially available from QIAGEN), using genome DNA extracted according to the standard protocol associated with the reagent as a template, and using the primer sets of SEQ ID NOs: 7, 8, 10, and 11, amplification by PCR, the hybridization reaction and labeling on the substrate, and fluorescence measurement were performed. In this case, primers in which the 5' ends of SEQ ID NOs: 8 and 11 were modified with biotin via a linker were used.

TaKaRa Multiplex Assay Kit Ver. 2 (commercially available from Takara Bio Inc.) was used for amplification. Each primer was added at a final concentration of 0.2 µM and the template genome DNA was added at 10 µg/L, a 2×Reaction buffer in TaKaRa Multiplex Assay Kit Ver. 2 was 1×, 1U of Multiplex Enzyme Mix was used, and the template DNA and the like were mixed to prepare a PCR reaction solution. T100 Thermal Cycler (commercially available from Bio-Rad) was used as the nucleic acid amplification device. After the amplification reaction was completed, the heat denaturation reaction was performed with the same instrument before performing the hybridization reaction on the substrate.

For the reagent used for the hybridization reaction on the substrate, a solution containing a substrate 1 on which the probe of SEQ ID NO: 9 was immobilized and a substrate 2 on which the probe of SEQ ID NO: 12 was immobilized, a saline-sodium-phosphate-EDTA Buffer (SSPE-buffer), and a heat-denatured PCR reaction solution were mixed.

Streptavidin - Phycoerythrin (commercially available from PlexBio) was used as a reagent for labeling. For the hybridization reaction and labeling on the substrate, IntelliPlex 1000 πCode Processor (commercially available from PlexBio) was used as an instrument. PlexBio (registered trademark) 100 Fluorescent Analyzer (commercially available from PlexBio) was used as the fluorescence measurement device.

The reaction conditions used are as follows.
Amplification reaction conditions
   94°C: 30 seconds
   60°C: 60 seconds->94°C: 30 seconds (30 cycles)
   72°C: 600 seconds
Heat denaturation conditions
   95°C: 5 minutes->rapid cooling to 4°C
Hybridization conditions
   37°C: 20 minutes incubation
Labeling conditions
   37°C: 10 minutes incubation

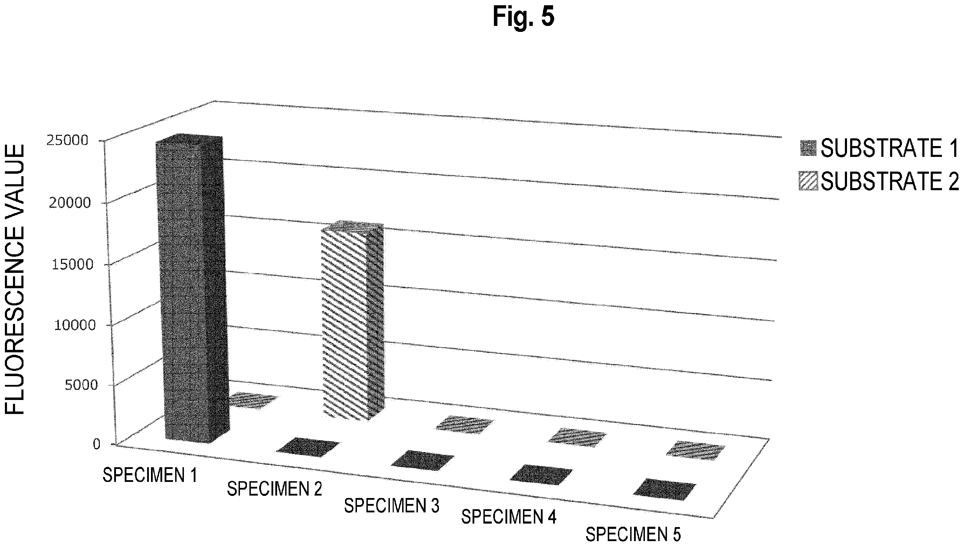

Fig. 5 shows the measurement results. Here, in Example 2, in response to a high background value of the substrate 1, the value when water (negative control) was used as a template was subtracted in Fig. 5.

When the specimen 1 was used as a template, fluorescence was observed on the substrate 1, and when the specimen 2 was used as a template, fluorescence was observed on the substrate 2. Based on this, in view of the results of Example 1, it was suggested that the specimen 1 could be *K. variicola.* In the verification 1 to be described below, the specimen 1 was subjected to whole genome analysis using a next-generation sequencer and bacterial strain identification by ANI, and as a result, it was found that the specimen 1 was classified as *K. variicola,* and it was clearly understood that, in combination with Example 1, by using the primer sets of SEQ ID NOs: 7, 8, 10, and 11, and the probes of SEQ ID NO: 9 and SEQ ID NO: 12, differentiation of *K. variicola* and *K. pneumoniae,* which could not be distinguished in the related art, would be possible.

In addition, for the specimens 3, 4, and 5, since no fluorescence was observed from any of the substrates, it was found that, when the primer sets of SEQ ID NOs: 7, 8, 10, and 11, and the probes of SEQ ID NO: 9 and SEQ ID NO: 12 were used, *K. aerogenes, K. oxytoca,* and *K. planticola,* which are other *Klebsiella spp.* bacteria, were not erroneously detected.

### Verification 1: whole genome analysis using next-generation sequencer for specimen 1 and bacterial strain identification by ANI

In bacterial strain identification using gene sequence information, a DNA-DNA hybridization (DDH) method has long been used, but an average nucleotide identity (ANI) method has taken the place of a standard method now.

ANI is a method of identifying bacterial strains based on the calculation results of which reference sequences registered in the database have a high sequence similarity to the sequence of whole genome to be analyzed.

### i) Picking bacteria up from isolated colonies and genome extraction

Bacteria were picked up from isolated colonies and the genome was extracted from the supernatant treated with phenol/chloroform/isoamyl alcohol (commercially available from NIPPON Genetics Co., Ltd.) using FastGene Gel/PCR Extraction Kit (commercially available from NIPPON Genetics Co., Ltd.) according to the standard protocol associated with the reagent.

### ii) Library preparation

A library was prepared using QIAseq FX DNA Library kit (commercially available from QIAGEN) according to the standard protocol associated with the reagent.

### iii) Sequencing reaction

Using MiSeq Reagent kit v3 600 cycles, according to the standard protocol associated with the reagent, a 300 bp×2 paired-end sequencing reaction was performed using MiSeq (commercially available from illumina) as an instrument.

### iv) Bacterial strain identification by ANI

For the raw data acquired from the instrument, using Trimmomatic version 0.38 for trimming, the sequence of the whole genome was created using SPAdes version 3.13.0 for de novo assembly. Using NCBI RefSeq of the National Center for Biotechnology Information as the database, bacterial strain identification by ANI was performed with BBTools.

The specimen 1 exhibited the highest similarity (ANI Value: 99.15%) with *K*. *variicola* in the database, and the runner-up was *K. pneumoniae* (ANI Value: 95.89%). Generally, in the bacterial strain identification by ANI, the threshold value for determining that bacterial strains were the same was 95 to 96% (Kim, M, HS Oh, SC Park, J Chun. 2014.Towards a taxonomic coherence between average gyrBleotide identity and 16S rRNA gene sequence similarity for species demarcation of prokaryotes. Int J Syst Evol Microbiol 64: 346-351.), and according to this threshold value, the specimen 1 was identified as *K. variicola.* From this, the validity of the results of Example 2 was also supported by the whole genome analysis and the bacterial strain identification by ANI.

### Detection on substrate using DNA probe targeting genome DNA of K. variicola and K. pneumoniae

### Design of primer and probe

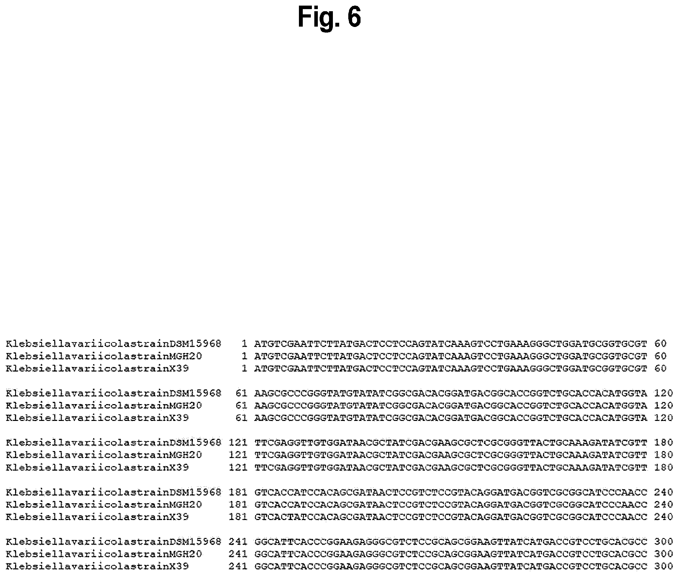

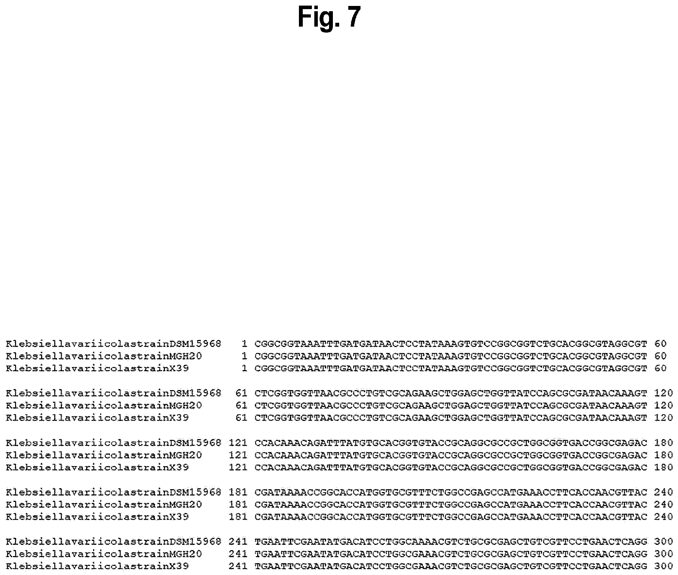

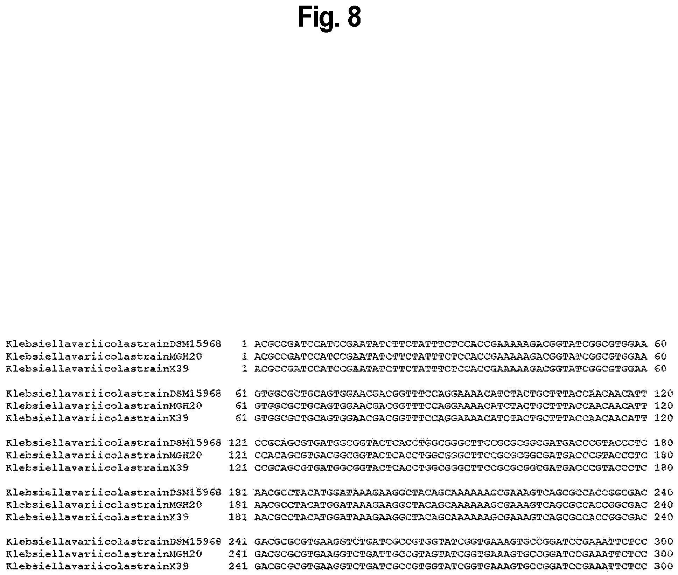

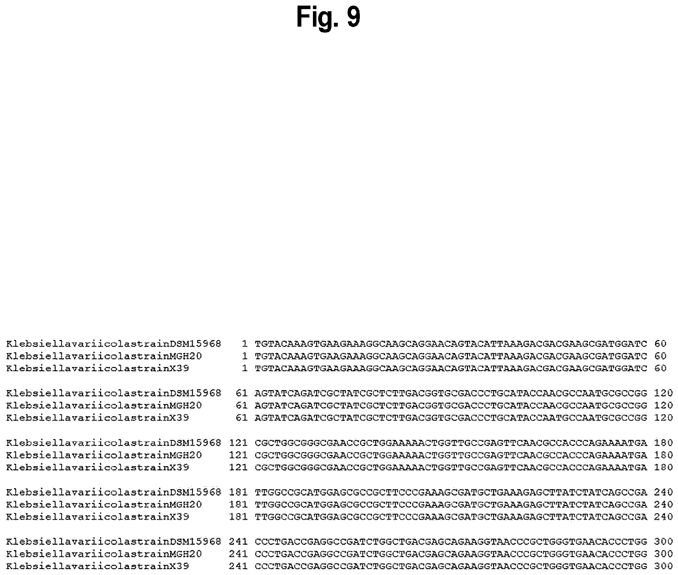

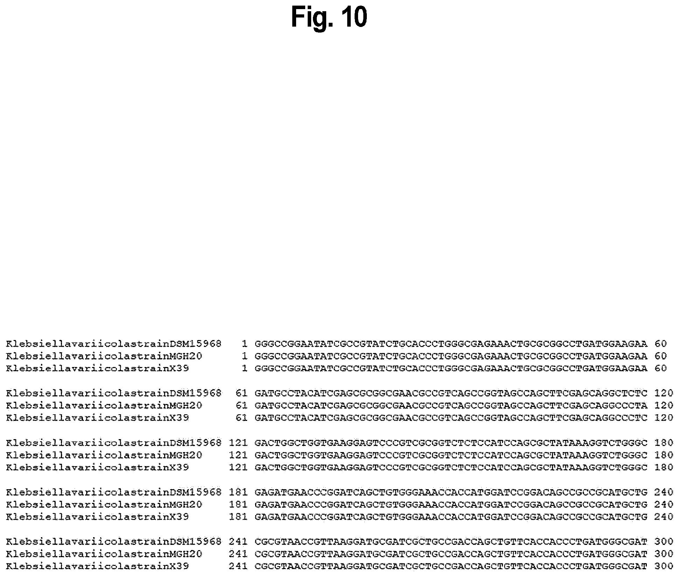

Primers and probes of SEQ ID NOs: 7 to 9 from the multiple alignment results of some gyrB genes (SEQ ID NOs: 1, 2, and 3) of *K. variicola* strain DSM 15968, strain MGH 20, and strain X39 shown in Fig. 1, SEQ ID NOs: 13 to 15 from the multiple alignment results of some gyrB genes (SEQ ID NOs: 1, 2, and 3) of *K. variicola* strain DSM 15968, strain MGH 20, and strain X39 shown in Fig. 6, SEQ ID NOs: 16 to 18 of the multiple alignment results of some gyrB genes (SEQ ID NOs: 1, 2, and 3) of *K. variicola* strain DSM 15968, strain MGH 20, and strain X39 shown in Fig. 7, SEQ ID NOs: 19 to 21 from the multiple alignment results of some gyrB genes (SEQ ID NOs: 1, 2, and 3) of *K. variicola* strain DSM 15968, strain MGH 20, and strain X39 shown in Fig. 8, SEQ ID NOs: 22 to 24 from the multiple alignment results of some gyrB genes (SEQ ID NOs: 1, 2, and 3) of *K. variicola* strain DSM 15968, strain MGH 20, and strain X39 shown in Fig. 9, and SEQ ID NOs: 25 to 27 from the multiple alignment results of some gyrB genes (SEQ ID NOs: 1, 2, and 3) of *K. variicola* strain DSM 15968, strain MGH 20, and strain X39 shown in Fig. 10 were designed. In addition, these were designated as assay sets 1 to 6.

**[Table 2]**

| | | |
|---|---|---|
| Assay set 1 | SEQ ID NO: 7: K. variicola primer | GGTGAACACCCTGGTTTC |
| | SEQ ID NO: 8: K. variicola primer | CGATATTCCGGCCCCATG |
| | SEQ ID NO: 9: K. variicola probe | GTTGCAGCAGTTTGAG |
| Assay set 2 | SEQ ID NO: 13: K. variicola primer | CTGCAAAGATATCGTTGTCAC |
| | SEQ ID NO: 14: K. variicola primer | GTGACAACGATATCTTTGCAG |
| | SEQ ID NO: 15: K. variicola probe | GAGGTTGTGGATAAC |
| Assay set 3 | SEQ ID NO: 16: K. variicola primer | GTGGTTAACGCCCTGTC |
| | SEQ ID NO: 17: K. variicola primer | GCCAGAAACGCACCATG |
| | SEQ ID NO: 18: K. variicola probe | GCGCGATAACAAAGTC |
| Assay set 4 | SEQ ID NO: 19: K. variicola primer | GTATCGGCGTGGAAGTG |
| | SEQ ID NO: 20: K. variicola primer | CGGCACCTTCACGGATAC |
| | SEQ ID NO: 21: K. variicola probe | GATGACCCGTACCC |
| Assay set 5 | SEQ ID NO: 22: K. variicola primer | GCGATGGATCAGTATCAGATC |
| | SEQ ID NO: 23: K. variicola primer | TTTCAGCAGCGCTTTCGG |
| | SEQ ID NO: 24: K. variicola probe | CGACCCTGCATACC |
| Assay set 6 | SEQ ID NO: 25: K. variicola primer | GCCTGATGGAAGAAGATG |
| | SEQ ID NO: 26: K. variicola primer | CTGTCCGGATCCATGGTG |
| | SEQ ID NO: 27: K. variicola probe | CGGCGAACGTCGTC |

### Nucleic acid amplification and detection

Amplification by PCR was performed using the genome DNA extracted from *K. variicola* strain JCM 12419 and *K. pneumoniae* strain JCM 1662^{T} as a template, and the primer sets of SEQ ID NOs: 7, 8, 10, and 11, SEQ ID NOs: 13, 14, 10, and 11, SEQ ID NOs: 16, 17, 10, and 11, SEQ ID NOs: 19, 20, 10, and 11, SEQ ID NOs: 22, 23, 10, and 11, or SEQ ID NOs: 25, 26, 10, and 11, and the hybridization reaction and labeling on the substrate, and fluorescence measurement were performed. In this case, primers in which the 5' ends of SEQ ID NOs: 8, 11, 14, 17, 20, 23, and 26 were modified with biotin via a linker were used.

TaKaRa Multiplex Assay Kit Ver. 2 (commercially available from Takara Bio Inc.) was used for amplification. Each primer was added at a final concentration of 0.2 µM and the template genome DNA was added at 10 µg/L, a 2×Reaction buffer in TaKaRa Multiplex Assay Kit Ver. 2 was 1×, 1U of Multiplex Enzyme Mix was used, and the template DNA and the like were mixed to prepare a PCR reaction solution. T100 Thermal Cycler (commercially available from Bio-Rad) was used as the nucleic acid amplification device. After the amplification reaction was completed, the heat denaturation reaction was performed with the same instrument before performing the hybridization reaction on the substrate.

For the reagent used for the hybridization reaction on the substrate, a solution containing the substrate 1 on which the probe of SEQ ID NO: 9 was immobilized, the substrate 3 on which the probe of SEQ ID NO: 15 was immobilized, the substrate 4 on which the probe of SEQ ID NO: 18 was immobilized, the substrate 5 on which the probe of SEQ ID NO: 21 was immobilized, and the substrate 6 on which the probe of SEQ ID NO: 24 was immobilized, a saline-sodium-phosphate-EDTA Buffer (SSPE-buffer), and a heat-denatured PCR reaction solution were mixed.

Streptavidin - Phycoerythrin (commercially available from PlexBio) was used as a reagent for labeling. For the hybridization reaction and labeling on the substrate, IntelliPlex 1000 πCode Processor (commercially available from PlexBio) was used as an instrument. PlexBio (registered trademark) 100 Fluorescent Analyzer (commercially available from PlexBio) was used as the fluorescence measurement device.

The reaction conditions used are as follows.
Amplification reaction conditions
   94°C: 30 seconds
   60°C: 60 seconds->94°C: 30 seconds (30 cycles)
   72°C: 600 seconds
Heat denaturation conditions
   95°C: 5 minutes-> rapid cooling to 4°C
Hybridization conditions
   37°C: 20 minutes incubation
Labeling conditions
   37°C: 10 minutes incubation

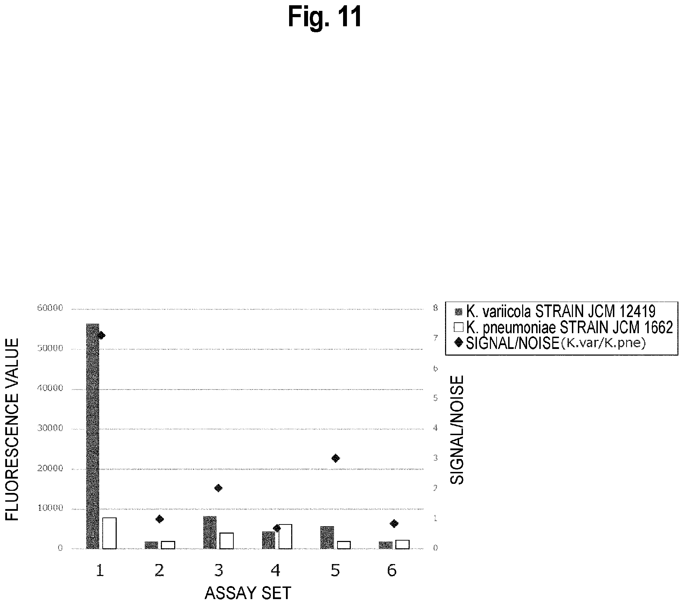

Fig. 11 shows the measurement results. In addition, the fluorescence value obtained when *K. variicola* strain JCM 12419 was used as a template was set as a signal and the fluorescence value obtained when *K. pneumoniae* strain JCM 1662^{T} was used as a template was set as noise, and the results of the calculated signal/noise of the assay sets are shown in Table 3.

**[Table 3]**

| Assay set | Fluorescence value of K. var | Fluorescence value of K. pne | Signal/noise (K. var/K. pne) |
|---|---|---|---|
| 1 | 56395 | 7896 | 7.1 |
| 2 | 1884 | 1871 | 1.0 |
| 3 | 8311 | 4046 | 2.1 |
| 4 | 4358 | 6256 | 0.7 |
| 5 | 5741 | 1891 | 3.0 |
| 6 | 1939 | 2276 | 0.85 |

It can be said that, as the value of signal/noise was larger, the assay set had a higher differentiating ability, and therefore, it was found that the assay set 1 had the highest differentiating ability, the assay set 5 had the next highest differentiating ability, and the assay set 3 had the third highest differentiating ability. It can be said that, as the value of signal/noise was closer to 1, the differentiating ability was low, or the assay set was indistinguishable, and it was found that the assay sets 2, 4, and 6 were assay sets having a low differentiating ability.

From this, it was found that, among the assay sets 1 to 6 created in six regions in the gyrB gene, the primers of SEQ ID NOs: 7 and 8 and the probe of SEQ ID NO: 9 included in the assay set 1 had the highest ability to distinguish between *K*. *variicola* and *K. pneumoniae.*

### [Sequence list]

International Application PU005488WO under the International Patent Cooperation Treaty, for detection of *Klebsiella spp.* bacteria, JP21013800 20210331-00140193152100703557 normal
20210331111115202103261133226220_P1AP101_PU_3.app

## Claims

1. A primer set for detecting the presence of one or more *Klebsiella spp.* bacteria selected from the group consisting of *Klebsiella variicola* and *Klebsiella pneumoniae* in a specimen, wherein the primer set consists of a first primer and a second primer that target the gyrB gene of *Klebsiella spp.* bacteria.

2. The primer set according to claim 1,
wherein the gyrB gene has a base sequence shown in any of SEQ ID NOs: 1 to 6, or has a base sequence in which one or several bases are deleted, substituted, inserted or added in the base sequence shown in any of SEQ ID NOs: 1 to 6, and encodes a GyrB protein.

3. The primer set according to claim 1 or 2,
wherein the target gyrB gene has a base sequence comprising at least 10 consecutive bases in the base sequence from the position 1887 to the position 2063 of the base sequence shown in any of SEQ ID NOs: 1 to 6.

4. The primer set according to any one of claims 1 to 3, which is for detecting the presence of *Klebsiella variicola,*
wherein the first primer has a base sequence shown in SEQ ID NO: 7 or a base sequence comprising at least 10 consecutive bases in a base sequence in which one or several bases are deleted, substituted, inserted or added in the base sequence shown in SEQ ID NO: 7 and has the same function as the base sequence shown in SEQ ID NO: 7.

5. The primer set according to any one of claims 1 to 4, which is for detecting the presence of *Klebsiella variicola,*
wherein the second primer has a base sequence shown in SEQ ID NO: 8 or a base sequence comprising at least 10 consecutive bases in a base sequence in which one or several bases are deleted, substituted, inserted or added in the base sequence shown in SEQ ID NO: 8 and has the same function as the base sequence shown in SEQ ID NO: 8.

6. The primer set according to any one of claims 1 to 5, which is for detecting the presence of *Klebsiella variicola,* and further comprises a third primer and a fourth primer targeting the gyrB gene of *Klebsiella pneumoniae.*

7. The primer set according to any one of claims 1 to 6,
wherein the third primer has a base sequence shown in SEQ ID NO: 10 or a base sequence comprising at least 10 consecutive bases in a base sequence in which one or several bases are deleted, substituted, inserted or added in the base sequence shown in SEQ ID NO: 10 and has the same function as the base sequence shown in SEQ ID NO: 10.

8. The primer set according to any one of claims 1 to 7,
wherein the fourth primer has a base sequence shown in SEQ ID NO: 11 or a base sequence comprising at least 10 consecutive bases in a base sequence in which one or several bases are deleted, substituted, inserted or added in the base sequence shown in SEQ ID NO: 11 and has the same function as the base sequence shown in SEQ ID NO: 11.

9. The primer set according to claim 1 or 2, which is for detecting the presence of *Klebsiella pneumoniae,*
wherein the first primer has a base sequence shown in SEQ ID NO: 10 or a base sequence comprising at least 10 consecutive bases in a base sequence in which one or several bases are deleted, substituted, inserted or added in the base sequence shown in SEQ ID NO: 10 and has the same function as the base sequence shown in SEQ ID NO: 10.

10. The primer set according to any one of claims 1, 2, and 9,
wherein the second primer has a base sequence shown in SEQ ID NO: 11 or a base sequence comprising at least 10 consecutive bases in a base sequence in which one or several bases are deleted, substituted, inserted or added in the base sequence shown in SEQ ID NO: 11 and has the same function as the base sequence shown in SEQ ID NO: 11.

11. The primer set according to any one of claims 1, 2, 9, and 10, further comprising
a third primer and a fourth primer that target the gyrB gene of *Klebsiella variicola.*

12. The primer set according to any one of claims 1, 2, and 9 to 11,
wherein the third primer has a base sequence shown in SEQ ID NO: 7 or a base sequence comprising at least 10 consecutive bases in a base sequence in which one or several bases are deleted, substituted, inserted or added in the base sequence shown in SEQ ID NO: 7 and has the same function as the base sequence shown in SEQ ID NO: 7.

13. The primer set according to any one of claims 1, 2, and 9 to 12,
wherein the fourth primer has a base sequence shown in SEQ ID NO: 8 or a base sequence comprising at least 10 consecutive bases in a base sequence in which one or several bases are deleted, substituted, inserted or added in the base sequence shown in SEQ ID NO: 8 and has the same function as the base sequence shown in SEQ ID NO: 8.

14. The primer set according to any one of claims 1 to 13,
wherein the primers are labeled with biotin.

15. A probe for detecting the presence of one or more *Klebsiella spp.* bacteria selected from the group consisting of *Klebsiella variicola* and *Klebsiella pneumoniae* in a specimen,
wherein the probe targets the gyrB gene of *Klebsiella spp.* bacteria.

16. The probe according to claim 15,
wherein the gyrB gene has a base sequence shown in any of SEQ ID NOs: 1 to 6, or has a base sequence in which one or several bases are deleted, substituted, inserted or added in the base sequence shown in any of SEQ ID NOs: 1 to 6, and encodes a GyrB protein.

17. The probe according to claim 15 or 16,
wherein the target gyrB gene has a base sequence comprising at least 10 consecutive bases in the base sequence from the position 1887 to the position 2063 of the base sequence shown in any of SEQ ID NOs: 1 to 6.

18. The probe according to any one of claims 15 to 17, which has a base sequence shown in SEQ ID NO: 9 or a base sequence comprising at least 8 consecutive bases in a base sequence in which one or several bases are deleted, substituted, inserted or added in the base sequence shown in SEQ ID NO: 9 and has the same function as the base sequence shown in SEQ ID NO: 9.

19. A kit comprising the primer set according to any one of claims 1 to 14, and the probe according to any one of claims 15 to 18.

20. A method for detecting the presence of *Klebsiella variicola* in a specimen, and in which the primer set according to any one of claims 1 to 9 is used.

21. The method according to claim 20, comprising
a step of differentiating between *Klebsiella variicola* and other *Klebsiella spp.* bacteria using first and second primers.

22. The method according to claim 20 or 21,
wherein the probe according to any one of claims 15 to 18 is additionally used.

23. The method according to any one of claims 20 to 22,
wherein the *Klebsiella spp.* bacteria distinguished from *Klebsiella pneumoniae* is one, two or more selected from the group consisting of *Klebsiella pneumoniae; Klebsiella michiganensis; Klebsiella quasipneumoniae*; *Klebsiella aerogenes; Klebsiella planticola; Klebsiella granulomatis; Klebsiella grimontii;* and *Klebsiella oxytoca.*

24. The method according to any one of claims 20 to 23,
wherein the *Klebsiella spp.* bacteria distinguished from *Klebsiella pneumoniae* is *Klebsiella pneumoniae,* and a fourth primer, a fifth primer and/or a probe that target the gyrB gene of *Klebsiella pneumoniae* are additionally used.

25. A method for detecting the presence of *Klebsiella pneumoniae* in a specimen, and in which the primer set according to any one of claims 1, 2, and 9 to 14 is used.

26. The method according to claim 25, comprising
a step of differentiating between *Klebsiella pneumoniae* and other *Klebsiella spp*. bacteria using first and second primers.

27. The method according to claim 26,
wherein the probe according to any one of claims 15 to 18 is additionally used.

28. The method according to any one of claims 25 to 27,
wherein the *Klebsiella spp.* bacteria distinguished from *Klebsiella pneumoniae* is one, two or more selected from the group consisting of *Klebsiella variicola; Klebsiella michiganensis; Klebsiella quasipneumoniae*; *Klebsiella aerogenes; Klebsiella planticola; Klebsiella granulomatis; Klebsiella grimontii;* and *Klebsiella oxytoca.*

29. The method according to any one of claims 25 to 28,
wherein the *Klebsiella spp.* bacteria distinguished from *Klebsiella pneumoniae* is *Klebsiella variicola,* and a fourth primer, a fifth primer and/or a probe that target the gyrB *gene* of *Klebsiella variicola* are additionally used.
